# EUROPEAN PATENT APPLICATION

(11) **EP 1 780 291 A1**
(43) Date of publication of application: **02.05.2007**
(21) Application number: 06122934.0
(22) Date of filing: 25.10.2006
(51) Int. Cl.: C12Q 1/68

(54) **Method of detecting specific fragments of DNA or RNA with the aid of a polymerase chain reaction**

(30) Priority: 26.10.2005 RU 2005132940
(71) Applicant: Institut Biokhimii I Genetiki Ufimskogo Nauchnogo Tsentra Ran, Ufa 450054 (RU)
(72) Inventor: Chemeris, Alexei Viktorovich, Ufa 450097 (RU); Nikonorov, Yury Mikhailovich, Ufa 450000 (RU); Romanenkova, Maiya Leonidovna, Ufa 450000 (RU); Chemeris, Dmitry Alexeevich, Ufa 450097 (RU); Garafutdinov, Ravil Rinatovich, Meleuz 453310 (RU); Magazova, Roza Azatovna, Ufa 450097 (RU); Maleev, Grigory Vladimirovich, Moscow 121351 (RU); Vakhitov, Vener Absatarovich, Ufa 450006 (RU); Vasilov, Raif Gayanovich, Moscow 119415 (RU)
(74) Representative: Henriksson, Dan Ragnar Mikael

(57) **Abstract**

A method of amplification with the aid of a polymerase chain reaction of specific fragments of DNA or RNA in real time is developed. A distinctive specificity of the proposed approach is detection of the results of PCR or RT-PCR in a special DNA amplifier provided with an optical module by registration of an increase of emission occurring due to transfer of fluorescent resonant energy from a donor dye to an acceptor dye or, in a second variant, by registration of a reduction of emission due to occurrence of the effect of quenching the emission of the dye by a universal quencher, the dyes included separately in the structure of the forward and reverse primers that do not have a secondary structure and are annealed on the target directly adjacent to each other or even slightly overlapping. This method may be recommended for use when carrying out DNA diagnosis in medicine, veterinary medicine, sanitary-epidemiological research, criminalistics, food industry, for detection of pathogens of dangerous infections, identification of criminals, detection of food products made from genetically modified organisms, determination of the quality of raw materials, etc.

## Description

The invention relates to molecular biology and biotechnology and is related to an analysis of DNA and/or RNA molecules. It may be used during the conduction of DNA diagnosis in medicine, veterinary medicine, sanitary-epidemiological research, criminalistics, food industry for detection of pathogens of dangerous infections, including possible bioterroristic attacks, identification of criminals, detection of food products made from genetically modified organisms, determination of the quality of raw materials, etc.

Existing methods of DNA diagnosis are based primarily on the amplification of specific fragments of DNA or RNA with the aid of a polymerase chain reaction (PCR) and modifications thereof. In recent times the so-called PCR in real time (RT-PCR), when detection of the desired product is conducted directly during amplification with the aid of special DNA amplifiers provided with an optical module, is the most widely used method. One of the important advantages of such an approach is the absence of the necessity of carrying out the step of electrophoretic separation of the reaction products, which step presumes the opening of test tubes and manipulation of their content in air, as a result of which the working zone of the rooms may be contaminated with the PCR products - amplicons, presented by billions of copies. This may result in the obtainment during subsequent analyses of falsely-positive results, which may occur since the new reaction mixtures may be initially contaminated in an aerosol manner with amplicons formed during preceding positive reactions and circulating in the air medium. In addition to this, the possibility of a procedure without electrophoretic analysis significantly reduces the time necessary for the whole procedure. Another important advantage is the possibility to obtain, with the aid of RT-PCR, an exact quantitative determination of the number of copies of one or another target, for example, the content of genetically modified ingredients or any other admixtures in raw material or food products.

At the present time, there are quite a few schemes for detecting desired products in RT-PCR, which may be conditionally divided into highly-specific and lowly-specific. The lowly-specific should include those which make it possible to detect only the appearance in the reaction mixture of new DNA fragments as such, independent of their nucleotide sequence, through the emission of intercalating dyes, such as ethidium bromide [Higuchi et al., 1992; 1993], SYBR Green I [Wittwer et al., 1997], BEBO [Bengtsson et al., 2003], LCGreen [Wittwer et al., 2003; Zhou et al., 2004], which in a complex with DNA emit significantly stronger as compared with their background fluorescence. A doubtful advantage of these methods is that they are the cheapest.

The group of lowly-specific methods should also include variants of RT-PCR that are based on a change of the fluorescence of one of the primers, which occurs during the amplification and operation of double-chain DNA fragments. The reason for their low or at least reduced specificity is that a change of the emission in this case does not in any manner depend on the second primer, or more exactly, on the place where it is annealed. That is, as a result of the nonspecific annealing (at such a distance from each other that the building of new DNA fragments will still be possible) of both primers or of only one marked primer as the "direct" and "reverse" right away in two places, amplification of the already unspecific DNA fragment in this case will take place anyway, but in the course of the increase or reduction of the fluorescence it will not be possible to state that it is not specific. So, for example, variants of RT-PCR with the positioning in one of the primers of a dye and quencher, which in the process of amplification should spatially separate under the action of thermally stable restriction endonuclease, since at first a site of recognition of this enzyme is present in the primer sequence [Cairns et al., 2004], or with the presence of fluorochrome and a closely positioned thereto unnatural nitrogeneous base isoC in the structure of the primer, which in the process of amplification results in the introduction of a complementary thereto isoG carrying a universal quencher into another DNA chain [Johnson et al., 2004], cannot be regarded as highly-specific methods, in spite of the fact that in the first case of RT-PCR with the participation of a modified primer with a restriction site this results in an increase of fluorescence, and in the second - to a decrease thereof. An additional disadvantage of some lowly-specific methods is furthermore the extreme complexity of the primer structures of the Sunrise [Nazarenko et al., 1997], Scorpion [Thelwell et al., 2000; Solinas et al., 2001; Huang et al., 2004], Cyclicon [Kandimalla, Agrawal, 2000] and other types. Wherein a negative moment is also that there are additional nucleotide sequences contained in such a primer in addition to a region annealing in the desired sequence, which may also have a significant affect on the specificity of the annealing.

The proposed RT-PCR method, designed for use of a hybridization universal UT probe [Zhang et al., 2003], has some advantage in the aspect that the probe may in fact be just one for almost all of the cases, which significantly reduces expenses related to RT-PCR, but the place for annealing it is positioned on one of the specially lengthened for this purpose primers and only in view of this the instant method should be recognized as being lowly-specific. There was also some intermediate variant proposed for positioning one of the dyes on the primer, the other on the hybridization probe [Rasmussen et al., 2003]. The authors called their nonstandard method PriProET, but positioned the donor and acceptor dyes at an excessively large distance from each other and made the size of the amplicon unjustifiably large, which brought to nothing all the advantages of the instant still highly-specific approach.

The methods of detection of the desired product in RT-PCR, which are based on use of a hybridization probe, should be considered to be highly specific in view of the fact that in addition to the two specifically annealing primers, such systems also have a probe, marked in a corresponding manner, that confirms by its hybridization with the amplicon the identity of the product with the expected. Among such probes, the most widely spread was the method of so-called TaqMan detection, which is based on the use of 5'-exonuclease activity of the Taq polymerase, under the effect of which destruction of the hybridization probe annealed on the amplicon and detachment of the fluorochrome, which begins emission, take place, while when it is in the structure of the probe together with the quencher its emission is extinguished [Lee et al., 1993; Livak et al., 1995]. Another highly-specific method of RT-PCR is based on the use of so-called molecular signal lights (Beacon), which are an oligonucleotide hairpin with dye and quencher positioned on the 5'- and 3'-ends [Tyagi, Kramer, 1996; Tyagi et al., 2000, Horejsh et al., 2005]. When a region of single-chain DNA, that is complementary to this probe, is formed in the process of amplification, opening the hairpin and annealing it take place accompanied by spatial distancing of the dye from the quencher, which results in an increase of fluorescence.

The main drawback of all of these systems is their complexity and the necessity to use a greater number of components in the RT-PCR than are present in an ordinary PCR, wherein in addition to the initial DNA, DNA polymerase and desoxy-nucleotide triphosphates, usually only two primers are sufficient for diagnostic purposes. The use of hybridization probes, carrying therein two, sometimes three modifications (significantly increasing their cost), also presumes the presence of a specific region, serving as a target for such a probe, between the places of annealing the primers, which also inevitably results in that the size of the amplicon increases and usually exceeds 60 - 70 bp and even more.

The object of the invention is to significantly simplify, accelerate and reduce the cost of obtaining the results of RT-PCR with maintenance of high specificity of the reaction.

The essence of the invention consists in that highly-specific detection of desired products of PCR is carried out in a special DNA amplifier provided with an optical module, wherein in the process of the reaction of amplification itself, the increase of the amount of desired products is registered according to an increasing intensity of the emission of an acceptor dye after the transfer of fluorescent resonance energy thereto from a donor dye or, in another variant, according to a decreasing emission of the fluorescent dye as a result of the effect of a quencher, separately included in the structure of quite ordinary (if their modification in the form of fluorescent dyes is not taken into account), deprived of a secondary structure, forward and reverse primers, while either hybridization probes of the TaqMan or Beacon and other types or complex primer constructions of the Sunrise, Scorpion or Cyclicon type, which have a complex secondary structure or intercalating dyes characterized by very low specificity are used for this purpose in the RT-PCR methods used at the present time.

The main distinction of our method is that there is no need of any additional structures in the form of hybridization probes, which make this process significantly more expensive and furthermore slow it down somewhat. In our variant the standard 25 cycles are accomplished in less than 25 min, since the size of the amplicon is only about 35 - 50 bp in view of the fact that the sections of the nucleotide sequence of the detected fragment of DNA or RNA, which are selected as places for annealing the forward and reverse primers, are located adjacent to each other or may even partially overlap. The high specificity of our RT-PCR method also called "UFA" (Universal Fluorescent Amplification) is provided in that the donor dye and the acceptor dye or, in another variant, the dye and quencher, are included in the structure of different (forward and reverse) primers. On the one hand this eliminates possible non-specific amplification (more precisely, its detection) only from one of the primers, and on the other hand - requires annealing of both primers in direct proximity to each other, which in accordance with the theory of probability is a virtually improbable event for a section that is not selected as the detected region.

Known technical solutions (analogs) that have features similar to the features distinguishing the claimed solution over the prototype were not found. It is the opinion of the authors that the claimed solution conforms with the "essential distinctions" criterion.

The proposed method of amplification UFA with the aid of a polymerase chain reaction of specific fragments of DNA or RNA together with their detection in the real time regimen is illustrated by the following examples.

### Example 1. Selection of oligonucleotide primers

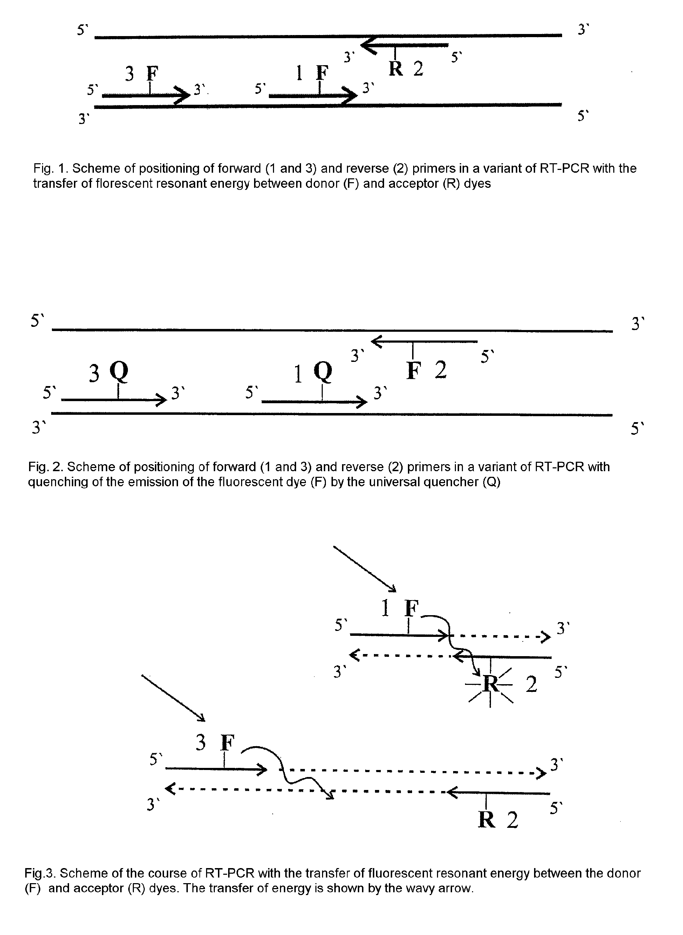

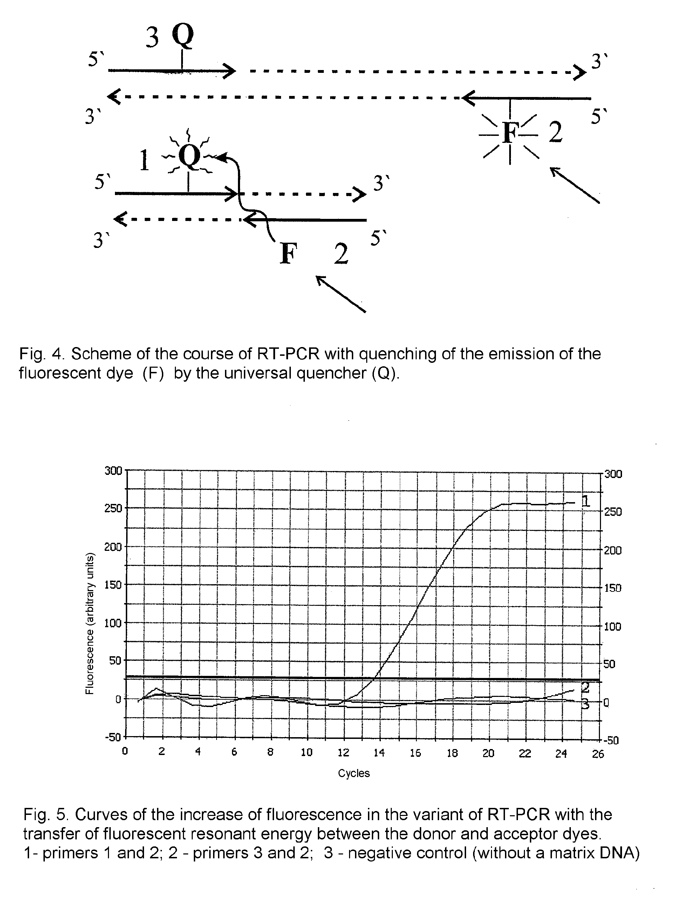

The selection of oligonucleotide primers is carried out with the expectation that they would provide specificity of the reaction and limit the fragment of nucleotide sequence of the DNA (or RNA) to a size of 35-50 pairs of nucleotides (or nucleotides) respectively. The following circumstance should be taken into account during the selection of the primers - that in the process of synthesis of the oligonucleotide primers (or postsynthetic) they could be marked with corresponding fluorescent dyes and quenchers, characterized by suitable wavelengths of excitation and emission and thus composing either a donor/acceptor pair (Fig. 1) or a dye/quencher pair (Fig. 2) and as a result of amplification be spaced from each other in a desired double-chain product at a distance where the transfer of fluorescent resonant energy (Fig. 3) or quenching (Fig. 4) is effectively detected. Primers for negative-positive control were selected, taking into account that the distance between the donor dye and the acceptor dye or dye and quencher would not make it possible in the first case to carry out transfer of the fluorescent resonant energy, and in the second - the effect of quenching would not be present, but wherein the corresponding RT-PCR product would be produced in both cases.

### Example 2. The conduction of preliminary DNA amplification in order to determine the melting temperature of a desired product

A reaction mixture with a volume of 25 µl comprises a buffer (40 mM Tris-HCl pH 8.0, 2.5 mM MgCl₂, 25 mM KCl); 20 fMol DNA; 1 unit of activity Taq DNA polymerase; 0.5 pMol of each of two primers, each marked with its fluorochrome (with a donor and acceptor or dye and quencher) and a corresponding amount of distilled water. RT-PCR was conducted in a DNA amplifier of the iCycler iQ model (Bio-Rad Laboratories, US) under the following conditions: denaturation of double-chain DNA in the first cycle was carried out at 95°C for 30 sec, then the primers were annealed and lengthened - 45°C, 10 sec with registration of the fluorescence at the end of this stage, denaturation of the desired product - 90°C, 10 sec, number of cycles - 25. Control of the production of the desired product was conducted, when it was presented in the form of a double-chain molecule, by registering an increase of the emission of the acceptor dye after its excitation with the fluorescent resonant energy of the donor dye or, in another variant - a decrease of the emission of the dye as a result of the quenching effect, which are present at this stage. The annealing temperature of the primers was varied somewhat in different experiments depending on the GC-structure of the used oligonucleotides. Determination of the melting temperature of the desired product of DNA amplification was carried out in the same DNA amplifier as provided for by the corresponding protocol of the program accompanying the device. After completion of the amplification step, detection of the temperature of transfer of the amplicon from the double-chain state to the single-chain was found by establishing the relationship of the negative value of the first derivative of fluorescence in respect to temperature, thus obtaining a differential curve of melting and experimentally determining the melting temperature of the desired product. This information is necessary for the selection of a temperature that is sufficient for sure denaturation of amplicons instead of the classical 95°C, since in that case the time for the whole amplification procedure is reduced in addition to other advantages. Taking into account that the average rate of heating and cooling the reaction blocks in DNA amplifiers of different models usually varies from 2 to 3°C per second, from 12 to 15 seconds may be saved in each cycle. Due to this, with a larger number of cycles, the working capacity of the enzyme is lengthened in view of the reduction of the time it is at temperatures above the optimum, thus on the whole providing more reliable amplification.

### Example 3. Preparation of cDNA

In order to amplify RNA comprising material with the aid of RT-PCR with an enzyme of reverse transcriptase (RT-RT-PCR), the step of building according to the RNA matrix of a complementary DNA with the aid of this enzyme is necessary. Preliminarily, 0.5 µg of RNA and 0.001 o.e. of oligonucleotide 2, serving here as a primer, were mixed in a microcentrifuge tube. The mixture was heated to 85°C and was allowed to slowly cool to 30°C. A reverse transcription reaction was carried out at 42°C during 10 min in a reaction volume of 20 µl of the following composition - 50 mM Tris-HC1 pH 8.3, 10 mM MgCl₂, 10 mM DTT, 0.5 mM spermidine, 0.5 mM of each of dNTF and 5 units of activity of AMV-revertase.

### Example 4. Carrying out RT-PCR or RT-RT-PCR

PCR was carried out in 25 µl of a reaction mixture comprising a buffer (40 mM Tris-HCl pH 8.0, 2.5 mM MgCl₂, 25 mM KCl); 20 fMol DNA; 1 unit of activity Taq DNA polymerase; 0.5 pMol of each of 2 primers marked each with its own fluorochrome (donor and acceptor or dye and quencher) and a corresponding amount of distilled water. RT-PCR was carried out in a DNA amplifier of the iCycler iQ model under the following conditions: denaturation of a double-chain DNA in the first cycle was conducted at 95°C for 30 sec, followed by annealing the primers and their lengthening - 45°C, 10 sec with registration of the fluorescence at the end of this step, denaturation of the desired product - 80°C, 10 sec, the number of cycles - 25.

Denaturation of the desired product at a reduced temperature (80°C) for a brief period of time (10 sec) made it possible to reduce the duration of the reaction and reduce the time during which the DNA polymerase is in the critical temperature zone, since there is no necessity to heat the desired product to 95°C, because, as was determined in example 2, it denaturates at a lower temperature (75°C). The annealing temperature of the primers and also the melting temperature of the desired products varied somewhat in different experiments depending on the GC composition of the used oligonucleotides. The second variant of RT-PCR with primers marked in one case by a donor dye and in the second by a quencher was conducted in a similar manner. As control, PCR was conducted without the addition of the studied DNA (or cDNA), and also with the addition of DNA, which does not comprise a region complementary to the used oligonucleotides. For additional negative-positive control, the region of the nucleotide sequence was selected that was intended to be the location for annealing primer 3, was positioned from the annealing point of primer 2 at a significant distance, and through which fluorescent resonant energy is not transmitted and the effect of extinguishing is also absent (Figs. 1 - 4). Curves of an increase of the fluorescence of the desired product, limited by primers 1 and 2, and the absence of a similar rise for the pair of primers 3 and 2 in the variant with donor and acceptor dyes are seen in Fig. 5.

### Example 5. Electrophoretic control of RT-PCR products (optional)

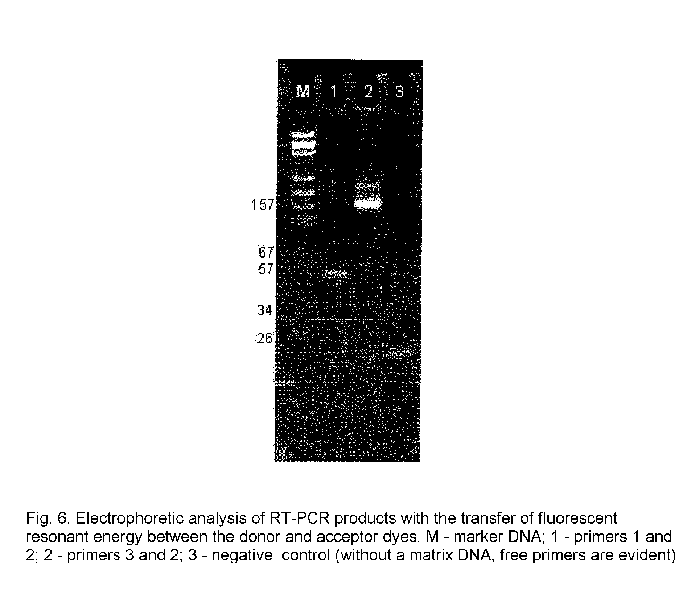

Electrophoretic control (optional) of the RT-PCR products was conducted with the purpose of visually detecting the desired product. Separation of the RT-PCR products was carried out in an 8% polyacrylamide gel in a tris-acetate buffer pH 7.8 in undenaturated conditions at a voltage gradient of 4V per cm of gel length in a vertical type device during 4 hours. After completion of electrophoresis, the gel after being colored with ethidium bromide was photographed in a photo-documentary system - Gel Camera System (UVP, Inc., US). As may be seen from Fig. 6, the desired product, registered during the RT-PCR, has the expected size of 42 bp, while the negative-positive control in the form of a strip of 110 bp size did not show a change of the fluorescence signal during the RT-PCR, but if dyed with ethidium bromide is quite evident. In view of the presence in the composition of the amplicons of two fluorochromes, their mobility is somewhat less than it was in the case of unmodified fragments of DNA of similar size.

### Literature taken into account

1. Bengtsson M., Karlsson H.J., Westman G., Kubista M. A new minor groove binding asymmetric cyanine reporter dye for real-time PCR // Nucl. Acids Res. 2003. V.31. e45.
2. Cairns M.J., Turner R., Sun L.Q. Homogeneous real-time detection and quantification of nucleic acid amplification using restriction enzyme digestion // Biochem. Biophys. Res. Comm. 2004. V.318. P. 684-690.
3. Higuchi R., Dollinger G., Walsh P.S., Griffith R. Simultaneous amplification and detection of specific DNA sequences // Biotechnology. 1992. V.10. P.413-417.
4. Higuchi R., Fockler C., Dollinger G., Watson R. Kinetic PCR analysis: real-time monitoring of DNA amplification reactions //Biotechnology. 1993. V.11. P.1026-1030.
5. Horejsh D., Martini F., Poccia F., Ippolito G., Di Caro A., Capobianchi M.R. A molecular beacon, bead-based assay for the detection of nucleic acids by flow cytometry // Nucleic Acids Res. 2005. V.33. e13.
6. Huang Y., Kong D., Yang Y., Niu R., Shen H., Mi H. Real-time quantitative assay of telomerase activity using the duplex scorpion primer // Biotechnol. Lett. 2004. V.26. P. 891-895.
7. Johnson S.C., Sherrill C.B., Marshall D.J., Moser M.J., Prudent J.R. A third base pair for the polymerase chain reaction: inserting isoC and isoG // Nucleic Acids Res. 2004. V.32. P. 1937-1941.
8. Kandimalla E.R., Agrawal S. "Cylicons" as hybridization-based fluorescent primer-probes: Synthesis, properties and application in real-time PCR // Bioorg. Med. Chem. 2000. V.8, P.1911-1916.
9. Lee L.G., Connell C.R., Bloch W. Allelic discrimination by nick-translation PCR with fluorogenic probes // Nucl. Acids Res. 1993. V.21. P.3761-6.
10. Livak K.J., Flood S.J., Marmaro J., Giusti W., Deetz K. Oligonucleotides with fluorescent dyes at opposite ends provide a quenched probe system useful for detecting PCR product and nucleic acid hybridization // PCR Methods Appl. 1995. V.4. P.357-362.
11. Nazarenko I.A., Bhatnagar S.K., Hohman R.J. A closed tube format for amplification and detection of DNA based on energy transfer // Nucleic Acids Res. 1997. V.25. P.2516-2521.
12. Rasmussen R.B., Uttenthal A., de Stricker K., Belak S., Storgaard T. Development of a novel quantitative real-time RT-PCR assay for the simultaneous detection of all serotypes of foot-and-mouth disease virus // Arch. Virol. 2003. V.148. P.2005-2021.
13. Solinas A., Brown L.J., McKeen C., Mellor J.M., Nicol J., Thelwell N., Brown T. Duplex Scorpion primers in SNP analysis and FRET applications // Nucleic Acids Res. 2001. V.29. E96.
14. Thelwell N., Millington S., Solinas A., Booth J., Brown T. Mode of action and application of Scorpion primers to mutation detection // Nucleic Acids Res. 2000. V.28. P.3752-3761.
15. Tyagi S., Kramer F.R. Molecular beacons: probes that fluoresce upon hybridization // Nat. Biotechnol. 1996. V.14. P.303-308.
16. Tyagi S., Marras S.A., Kramer F.R. Wavelength-shifting molecular beacons // Nat. Biotechnol. 2000. V.18. P. 1191-1196.
17. Wittwer C.T., Herrmann M.G., Moss A.A., Rasmussen R.P. Continuous fluorescence monitoring of rapid cycle DNA amplification // Biotechniques. 1997. V.22. P.130-131, 134-138.
18. Wittwer C.T., Reed G.H., Gundry C.N., Vandersteen J.G., Pryor R.J. High-resolution genotyping by amplicon melting analysis using LCGreen // Clin. Chem. 2003. V.49. P.853-860.
19. Zhang Y., Zhang D., Li W., Chen J., Peng Y., Cao W. A novel real-time quantitative PCR method using attached universal template probe // Nucleic Acids Res. 2003. V.31. e123.
20. Zhou L., Myers A.N., Vandersteen J.G., Wang L., Wittwer C.T. Closed-tube genotyping with unlabeled oligonucleotide probes and a saturating DNA dye // Clin. Chem. 2004. V.50. P.1328-1335.

## Claims

1. A method of amplifying specific fragments of DNA or RNA with the aid of a polymerase chain reaction in real time, **characterized in that** the use of hybridization probes therein is not necessary, but oligonucleotide primers are used that do not form secondary structures, that do carry fluorescent dyes in the structure thereof and that anneal on a target directly adjacent to each other or even slightly overlapping, thus providing a fluorescent resonant transfer of energy between a donor dye and an acceptor dye, which dyes are respectively included in the structure of forward and reverse primers and wherein detection of the accumulation of desired products of amplification is carried out by registration of an increase of emission of an acceptor fluorochrome.

2. The method according to claim 1, **characterized in that** the structure of the forward and reverse primers includes a fluorescent dye and a universal quencher, respectively, and detection of the accumulation of desired products of amplification is carried out by registration of a reduction of emission of the fluorochrome due to an occurring quencher effect.
